# EUROPÄISCHE PATENTSCHRIFT

(11) **EP 0 590 503 B1**
(45) Veröffentlichungstag und Bekanntmachung des Hinweises auf die Patenterteilung: **09.05.2001**
(21) Anmeldenummer: 93115302.7
(22) Anmeldetag: 23.09.1993
(51) Int. Cl.: G01N 33/36, G01N 27/22, G01B 7/12

(54) **Kapazitiver Sensor zur Erfassung von Masse- und/oder Durchmesserschwankungen von langgestrecktem textilem Prüfgut**
Capacitive sensor for the determiniation of variations in mass and/or diameter of a stretch of textile sample
Détecteur capacitif pour la détermination de variations dans la masse et/ou le diamètre d'un étalon allongé en textile

(30) Priorität: 01.10.1992 CH 306692
(43) Veröffentlichungstag der Anmeldung: 06.04.1994
(73) Patentinhaber: ZELLWEGER LUWA AG, 8610 Uster (CH)
(72) Erfinder: Hildebrand, Niklaus, CH-8636 Wald (CH)

(56) Entgegenhaltungen:
- EP-A- 0 197 763
- EP-A- 0 401 600
- GB-A- 2 020 816
- US-A- 3 922 601
- US-A- 4 006 411

## Beschreibung

Die vorliegende Erfindung betrifft einen kapazitiven Sensor zur Erfassung von Masse- und/oder Durchmesserschwankungen von langgestrecktem textilem Prüfgut, mit eine Messzone bildenden plattenförmigen Elektroden, welche einen zum Durchlauf des Prüfguts vorgesehenen Messschlitz begrenzen.

Derartige Sensoren weisen einen sogenannten Formeffekt und einen sogenannten Positionseffekt auf. Formeffekt bedeutet, dass Prüfgut von nicht exakt zylindrischem Querschnitt in Abhängigkeit von seiner Querlage im Messschlitz unterschiedlich starke Signale erzeugt. Positionseffekt bedeutet, dass Material beliebigen Querschnitts in Abhängigkeit von seiner Position zwischen den Messelektroden unterschiedlich starke Signale erzeugt. Bei optischen Sensoren, wo der Formeffekt besonders stark ist, versucht man diesen dadurch zu beherrschen, dass man das Prüfgut mit zwei zueinander gekreuzten Lichtbündeln abtastet. Bei kapazitiven Sensoren, bei denen der Formeffekt wesentlich geringer, der Positionseffekt aber wesentlich grösser ist als bei den optischen, hat man bisher versucht, eventuelle Abweichungen des Prüfguts vom zylindrischen Querschnitt durch Rotation des Prüfguts um dessen Achse zu egalisieren und eine genaue Positionierung durch aufwendige Führungen für das Prüfgut ausserhalb der Messzone zu erreichen. Diese Methoden sind mit steigenden Anforderungen an die Messgenauigkeit immer unbefriedigender und sie stellen auch hohe Anforderungen an die Abzugs- und Transportmittel für das Prüfgut.

Aus der US-A-4,006,411 und der US-A-3,922,601, die den nächstliegenden Stand der Technik angeben, sind Garnführungen für einen Messkopf mit einem Messschlitz bekannt, die in Längs- oder Bewegungsrichtung des Garns gesehen, am Eintritt und am Austritt des Messschlitzes angeordnet sind. Mit diesen Garnführungen, die ausserhalb der Messzone an zwei beabstandeten Stellen liegen, soll das Garn im Messschlitz zentriert werden oder es soll der Raum für seitliche Auslenkungen des Garns verringert werden.

Beide Lösungen haben aber den Nachteil, dass das Garn in der Messzone trotzdem eine beliebige Lage einnehmen kann, beispielsweise indem in der Messzone Schwingungsbäuche einer Resonanzschwingung im Garn sich bilden können. So kann das Garn nach wie vor seine Lage in der Messzone im Bereiche der gesamten Weite des Messschlitzes variieren, was bedeutet, dass der Einfluss des Positionseffektes und des Formeffektes dadurch nicht eingedämmt ist.

Durch die Erfindung sollen nun die bekannten kapazitiven Sensoren dahingehend verbessert werden, dass der Form- und der Positionseffekt ausgeschaltet oder zumindest auf eine vernachlässigbare Grösse von deutlich unter 10% reduziert wird.

Diese Aufgabe wird erfindungsgemäss dadurch gelöst, dass in der Messzone mechanische Mittel zur Verkleinerung der Weite des Messschlitzes vorgesehen sind. Diese Mittel sind vorzugsweise als Führungen ausgebildet.

Durch diese Mittel wird das Prüfgut in einem definierten Abstand von den Elektroden geführt und kann somit nicht mehr in die Randzonen des Messschlitzes gelangen. Damit werden aber die bisher zu nahe an die Elektroden gelangten Anteile des Prüfguts in einem grösseren Abstand von den Elektroden geführt, wodurch durch den Formeffekt und/oder den Positionseffekt verursachte Messfehler signifikant verringert werden. Denn gerade die Anteile des Prüfguts, die bisher bis in die Nähe der Elektroden gelangt waren, sind bis zu 20% überbewertet worden.

Im folgenden wird die Erfindung anhand eines Ausführungsbeispiels und der Zeichnungen näher erläutert; es zeigt:
- Fig. 1: eine Draufsicht auf eine einen kapazitiven Sensor aufweisende Messsonde für die Messung der Ungleichmässigkeit von Garnen oder Bändern,
- Fig. 2: eine Ansicht in Richtung des Pfeiles II von Fig. 1; und
- Fig. 3: ein erstes Detail von Fig. 1,
- Fig. 4: eine Ansicht in Richtung des Pfeiles IV von Fig. 3,
- Fig. 5: ein zweites Detail von Fig. 1; und
- Fig. 6: eine Ansicht in Richtung des Pfeiles VI von Fig. 5.

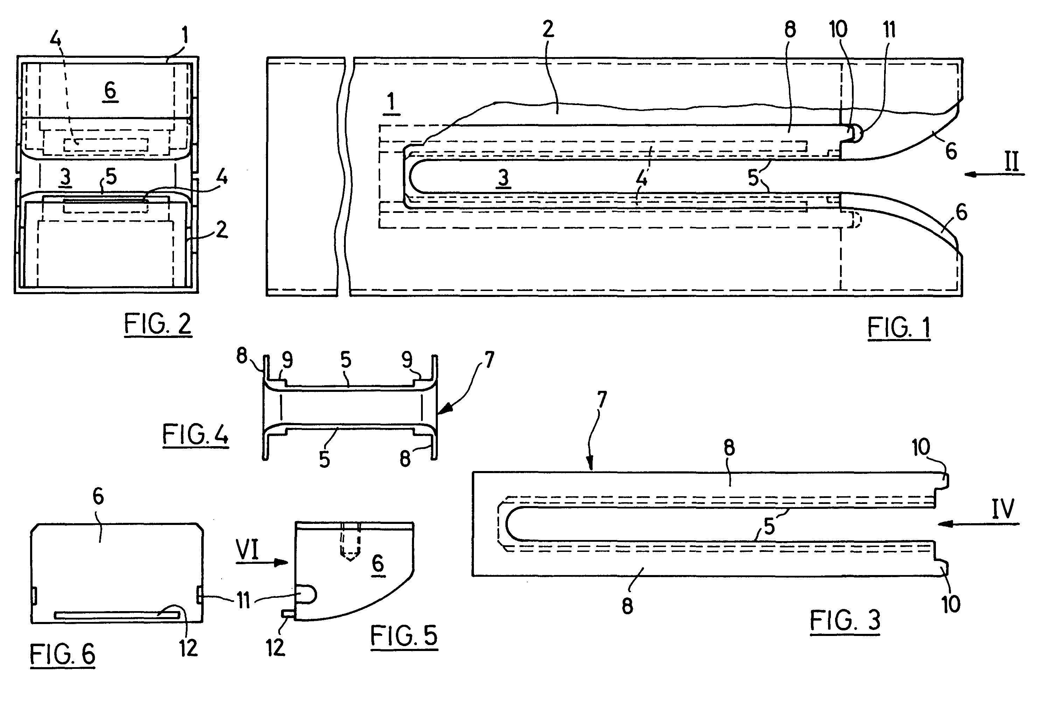

Fig. 1 zeigt eine Draufsicht auf eine Messsonde des Prüfgeräts MINI USTER, wie es von der Zellweger Uster AG seit Jahrzehnten für die Messung der Ungleichmässigkeit von Garnen oder Bändern direkt an der jeweiligen Maschine angeboten wird (MINI USTER und USTER sind registrierte Warenzeichen der Zellweger Uster AG). Die Messsonde besteht im wesentlichen aus einem in Fig. 1 teilweise aufgeschnittenen länglichen Gehäuse 1, das einen Halterungsblock 2 für zwei zu beiden Seiten eines Messschlitzes 3 angeordnete plattenförmige Metallelektroden 4 enthält, aus die Elektroden.4 gegen den Messschlitz 3 hin abdeckenden Führungen 5 aus nicht leitendem Material und aus zwei an die Elektroden 4 anschliessenden Backen 6, die eine trichterförmige Erweiterung zur Erleichterung des seitlichen Einführens des Prüfguts in den Messschlitz 3 bilden. Fig. 2 zeigt eine Ansicht gegen den Grund des Messschlitzes 3, wobei die in Fig. 1 untere Backe 6 weggelassen ist, die Fig. 3 und 4 zeigen zwei Ansichten des die Führungen 5 tragenden Bauteils und die Fig. 5 und 6 zeigen die in Fig. 1 obere Backe 6 in zwei Ansichten.

Die Elektroden 4 bilden in bekannter Weise einen kapazitiven Sensor zur Messung von Schwankungen der Masse und/oder des Durchmessers eines durch den Messschlitz 3 laufenden Prüfguts. Die Messsonden für den MINI USTER sind in verschiedenen Schlitzweiten zwischen 1 und 12 mm für Garne und Bänder erhältlich; die dargestellte Messsonde ist eine solche für Bänder und hat eine Schlitzweite von 12 mm.

Da der MINI USTER als bekannt vorausgesetzt wird (siehe dazu beispielsweise das USTER News Bulletin Nr. 28 vom Juni 1980, Kapitel 1.6 "Tragbarer Gleichmässigkeitsprüfer MINI USTER"), wird dieser hier nicht im Detail sondern nur in dem zum Verständnis der Erfindung erforderlichen Umfang beschrieben. Ausserdem wird ausdrücklich darauf hingewiesen, dass die Erfindung selbstverständlich nicht auf den MINI USTER beschränkt ist, sondern bei allen kapazitiven Sensoren zur Messung der Ungleichmässigkeit von textilem Prüfgut verwendet werden kann. Derartige Sensoren werden beispielsweise bei den bekannten Gleichmässigkeitsprüfern USTER TESTER und bei den elektronischen Garnreinigungsanlagen USTER AUTOMATIC, USTER POLYMATIC und USTER POLYGUARD verwendet.

Die Führungen 5, die verhindern, dass das Prüfgut zu nahe an die Elektroden 4 gelangen kann, dienen zur Vermeidung des Form- und des Positionseffekts. Der erstere besteht darin, dass nicht zylindrisches Prüfgut in Abhängigkeit von seiner Lage im Messschlitz 3 unterschiedlich starke Signale erzeugt. Positionseffekt bedeutet, dass Prüfgut von beliebigem Querschnitt in Abhängigkeit von seiner Position zwischen den Elektroden 4 ebenfalls unterschiedlich starke Signale erzeugt. Wenn man die lageabhängige Empfindlichkeit eines kapazitiven Sensors als Empfindlichkeitsänderung in Prozent als Funktion der Messschlitzweite aufträgt, dann erhält man eine Kurve in Form einer Badewanne. Diese Kurve zeigt, dass verglichen mit dem Signal eines in der Mitte des Messschlitzes 3 geführten Prüfguts, das Prüfgut innerhalb eines Abstandes von unter 10% vom Messschlitzrand um bis zu 20% überbewertet wird.

Wenn man nun durch die Führungen 5 den Messschlitz 3 auf 80% seiner Weite verringert und zu beiden Seiten jeweils einen Randbereich von 10% ausblendet, dann liegt die maximale Empfindlichkeitsänderung deutlich unter 10% und bei einem ausgeblendeten Randbereich von je etwa 15% der Schlitzweite unter 4%. So hat sich bei der dargestellten Messsonde mit einer Schlitzweite von 12 Millimetern ein Abstand der Führungen 5 von den Elektroden 4 von 2 Millimetern als optimal erwiesen.

Die Führungen 5 bilden vorzugsweise die beiden Schenkel eines U-förmigen Führungsteils 7, der in den Messschlitz 3 einschiebbar und in diesem fixierbar ist. Der Führungsteil 7 ist durch ein U-förmig gefaltetes, rinnenartiges Profilband gebildet, das einen die Führungen 5 bildenden Bodenteil und von diesem kragenartig abstehende Seitenstege 8 aufweist. Die letzteren dienen zur Fixierung des Führungsteils 7 am Messschlitz 3 in Laufrichtung des Prüfguts, das ist in Fig. 1 die Richtung senkrecht zur Zeichenebene. Wie insbesondere Fig. 2 entnommen werden kann, umgreifen die Seitenstege 8 des Führungsteils 7 zu diesem Zweck den Halterungsblock 2 für die Elektroden 4. Im Uebergangsbereich zwischen dem Boden und den Seitenstegen 8 des Profilbandes ist je eine Abstufung 9 vorgesehen, durch die eine Distanzierung der Führungen 5 von den Elektroden 4 erfolgt. Selbstverständlich können die Führungen 5 auch direkt auf den Elektroden 4 aufgebaut sein.

An ihren freien Enden laufen die Seitenstege 8 in je eine Fixiernase 10 aus, die in eine entsprechende Nut 11 der Backen 6 eingreift. Als weiteres Fixiermittel für den Führungsteil 7 weisen die Backen einen vorspringenden Steg 12 auf, der an der Hinterseite des die Führungen 5 bildenden Bodenteils des Profilbandes angreift. Auf diese Weise ist der Führungsteil 7 im Bereich seiner freien Enden durch die in die Nuten 11 eingreifenden Fixiernasen 10 und den die Führungen 5 fixierenden Steg 12 fest eingespannt.

Als Material für den Führungsteil 7 wird ein geeigneter Kunststoff verwendet, der insbesondere elektrisch nicht leitend sein darf und eine möglichst kleine Dielektrizitätskonstante haben sollte. Ausserdem muss der Führungsteil 7 mechanisch und chemisch stabil sein. Letzteres bedeutet, dass zwischen dem Prüfgut und den Führungen 5 keine chemischen Reaktionen stattfinden dürfen. In der praktischen Erprobung hat sich besonders Polymethylenoxid als geeignetes Material erwiesen. Dieses Material ist neben seinen guten Eigenschaften auch sehr preisgünstig und ist einfach zu verarbeiten.

## Patentansprüche

1. Kapazitiver Sensor zur Erfassung von Masse- und/oder Durchmesserschwankungen von langgestrecktem textilem Prüfgut, mit eine Messzone bildenden plattenförmigen Elektroden (4), welche einen zum Durchlauf des Prüfguts vorgesehenen Messschlitz (3) begrenzen, dadurch gekennzeichnet, dass in der Messzone mechanische Mittel (5) zur Verkleinerung der Weite des Messschlitzes vorgesehen sind.

2. Sensor nach Anspruch 1, dadurch gekennzeichnet, dass als mechanische Mittel Führungen (5) vorgesehen sind.

3. Sensor nach Anspruch 2, dadurch gekennzeichnet, dass die Führungen (5) aus einem elektrisch nicht leitenden Material mit kleiner Dielektrizitätskonstante bestehen.

4. Sensor nach Anspruch 3, dadurch gekennzeichnet, dass das Material der Führungen (5) durch Polymethylenoxid gebildet ist.

5. Sensor nach einem der Ansprüche 2 bis 4, dadurch gekennzeichnet, dass beide Führungen (5) über einen Mittelteil miteinander verbunden sind, womit ein einziger, im Messschlitz (3) fixierbarer Führungsteil (7) gebildet ist.

6. Sensor nach Anspruch 5, dadurch gekennzeichnet, dass der Führungsteil (7) aus einem flachen Band mit umgebogenen Seitenstegen (8) besteht und eine dem Messschlitz (3) angepasste Form aufweist, wobei die umgebogenen Seitenstege des Führungsteils die in Laufrichtung des Prüfguts vorderen und hinteren Kanten des Messschlitzes umgreifen und zur Fixierung des Führungsteils in der genannten Richtung dienen.

7. Sensor nach Anspruch 6, dadurch gekennzeichnet, dass der Führungsteil (7) gegen den Grund des Messschlitzes (3) gedrückt und in dieser Position fixiert ist.

8. Sensor nach Anspruch 5, dadurch gekennzeichnet, dass die Führungen (5) im Abstand von den Elektroden (4) angeordnet sind, und dass dieser Abstand 10% bis 30% der Weite des Messschlitzes (3) beträgt.

## Claims

1. Capacitive sensor for detecting fluctuations in the mass and/or diameter of elongated textile test product, the said sensor having plate-shaped electrodes (4) which constitute a measuring zone and which delimit a measuring slot (3) which is provided for the test product to run through, characterised in that mechanical means (5) for diminishing the width of the measuring slot are provided in the measuring zone.

2. Sensor according to claim 1, characterised in that guides (5) are provided as the mechanical means.

3. Sensor according to claim 2, characterised in that the guides (5) consist of an electrically non-conductive material with a small dielectric constant.

4. Sensor according to claim 3, characterised in that the material of the guides (5) is constituted by polymethylene oxide.

5. Sensor according to one of claims 2 to 4, characterised in that the two guides (5) are connected to one another via a central part, whereby a single guide part (7) which can be fixed in the measuring slot (3) is formed.

6. Sensor according to claim 5, characterised in that the guide part (7) consists of a flat strip with bent-over lateral webs (8) and has a shape which is adapted to the measuring slot (3), the bent-over lateral webs of the said guide part engaging round the edges of the measuring slot which are at the front and rear in the direction of running of the test product, and serving to fix the guide part in the aforesaid direction.

7. Sensor according to claim 6, characterised in that the guide part (7) is pressed against the base of the measuring slot (3) and fixed in that position.

8. Sensor according to claim 5, characterised in that the guides (5) are disposed at a distance from the electrodes (4), and that the said distance amounts to 10% to 30% of the width of the measuring slot (3).

## Revendications

1. Capteur capacitif pour détecter des variations de masse et/ou de diamètre d'un échantillon textile allongé, avec des électrodes (4) en forme de plaque formant une zone de mesure, qui délimitent une fente de mesure (3) prévue pour le passage de l'échantillon, caractérisé en ce que sont prévus dans la zone de mesure des moyens mécaniques (5) pour diminuer la largeur de la fente de mesure.

2. Capteur selon la revendication 1, caractérisé en ce que des guidages (5) sont prévus comme moyens mécaniques.

3. Capteur selon la revendication 2, caractérisé en ce que les guidages (5) sont constitués d'un matériau électriquement non conducteur d'une petite constante diélectrique.

4. Capteur selon la revendication 3, caractérisé en ce que le matériau des guidages (5) est constitué de polyméthylène oxyde.

5. Capteur selon l'une des revendications 2 à 4, caractérisé en ce que les deux guidages (5) sont reliés l'un à l'autre par une partie médiane par quoi est formée une seule partie de guidage (7) pouvant être fixée dans la fente de mesure (3).

6. Capteur selon la revendication 5, caractérisé en ce que la partie de guidage (7) est constituée d'une bande plate avec des nervures latérales recourbées (8) et a une forme adaptée à la fente de mesure (3), où les nervures latérales recourbées de la partie de guidage entourent les arêtes avant et arrière de la fente de mesure dans la direction d'avance de l'échantillon et servent é la fixation de la partie de guidage dans la direction indiquée.

7. Capteur selon la revendication 6, caractérisé en ce que la partie de guidage (7) est poussée contre le fond de la fente de mesure (3) et est fixée dans cette position.

8. Capteur selon la revendication 5, caractérisé en ce que les guidages (5) sont disposés à un écart des électrodes (4), et en ce que cet écart représente 10% à 30% de la largeur de la fente de mesure
